(19) Europäisches Patentamt
European Patent Office
Office européen des brevets

(11) Publication number: **0 013 713**
**B1**

(12) **EUROPEAN PATENT SPECIFICATION**

(45) Date of publication of patent specification: **11.04.84**

(51) Int. Cl.³: **C 11 D 1/34**, C 11 D 1/78, C 07 F 9/09

(21) Application number: **79104741.8**

(22) Date of filing: **28.11.79**

(54) **Phosphorus containing surface active agents and their preparation.**

(30) Priority: **30.11.78 US 965459**
**30.11.78 US 965458**
**30.11.78 US 965457**

(43) Date of publication of application:
**06.08.80 Bulletin 80/16**

(45) Publication of the grant of the patent:
**11.04.84 Bulletin 84/15**

(84) Designated Contracting States:
**AT BE CH DE FR GB IT LU NL SE**

(56) References cited:
**EP - A - 0 000 132**
**DE - A - 2 260 326**
**DE - A - 2 812 118**
**US - A - 3 825 628**
**US - A - 4 077 898**

**Handbook "Organic Phosphorous Compounds" by KOSOLAPOFF et al. Vol. 6**

(73) Proprietor: **Mona Industries**
**65-75 East 23rd Street**
**Paterson, New Jersey 07524 (US)**

(72) Inventor: **O'Lenick, Jr., Anthony J.**
**18-15 Marlot Avenue**
**Fairlawn, New Jersey 07410 (US)**
Inventor: **Mayhew, Raymond**
**5 Beekman Road**
**Summit, New Jersey 07901 (US)**

(74) Representative: **Werner, Hans-Karsten, Dr. et al,**
**Deichmannhaus am Hauptbahnhof**
**D-5000 Köln 1 (DE)**

**EP 0 013 713 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Phosphorus containing surface active agents and their preparation

The present invention relates to a novel process for the preparation of phosphorus-containing amphoteric quaternary and zwitterionic surface active agents, and to certain novel surface active agents per se.

## Background of the Invention

Quaternaries, betaines and certain substituted betaines are known in the art but the specific compounds made in accordance with the process of the instant invention have been found to be particularly useful. Thus, the compounds made by the present invention exhibit outstanding foaming, viscosity-building, wetting, cleansing, detergency, anti-static and emulsifying properties and are therefore useful in industrial applications calling for high performance surface active agents. The compounds are also highly stable species and are extremely well tolerated by human tissue, i.e., they exhibit exceptionally low ocular irritation and oral toxicity, and are therefore eminently suited and useful as surface active agents in personal care compositions.

Certain zwitterionic phosphorus-containing surface active agents and a process for the preparation thereof are disclosed in DE—A—2 260 326. These compounds have an alkyl group of 1 to 4 carbon atoms between the phosphorus and the amino group, which, however, cannot be substituted by a hydroxy group. Substances having a 2-hydroxypropyl group between the phosphorus group and the amino group cannot be made by reacting a cyclic phophite with an amino group as described in DE—A—2 260 326.

In "Organic Phosphorus Compounds" by G.M. Kosolapoff and L. Maier, edited by Wiley-Interscience, New York, 1973, volume 6, pages 232, 357, and 387 to 389, other zwitterionic compounds are disclosed which, however, do not have a 2-hydroxypropyl group between the phosphorus group and the amino group. Whenever they are produced by reacting an amine with a corresponding Hal-alkylene-phosphate ester, the hal is bromine, as chlorine is not reactive enough to react with the amine.

It now has been found that a group of novel phosphorus ester reactants of the formula

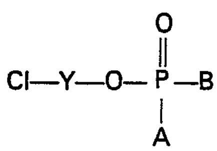

wherein
A is selected from OM, and OYCL,
B is selected from H, OM', and OYCL with the proviso that M' is the organic radical, and
Y is 2-hydroxypropyl,
can be made in a very easy and economic way and can be reacted very easily and with high yields with amines having more than 6 carbon atoms under specific conditions. The resulting surface active agents exhibit outstanding properties compared with those known up to now.

Subject of this invention, therefore, is a process for the production of phosphorus-containing surface active agents, which process comprises reacting a phosphorus ester reactant selected from phosphate di- and tri-esters and phosphite di-esters, having a labile halogen structure susceptible to nucleophilic attack, with an amine, characterized by the reaction of an amine having at least 6 carbon atoms for at least 3 hours with a phosphorus ester reactant of the formula:

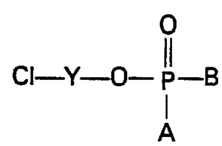

wherein
A is selected from OM, and OYCl,
B is selected from H, OM' and OYCl with the proviso that M' is an organic radical,
Y is 2-hydroxypropyl,
M and M' are individually an organic radical selected from alkyl or hydroxylalkyl of up to 6 carbon atoms, dihydroxyalkyl of up to 10 carbon atoms, glyceryl, cycloalkyl of up to 6 carbon atoms, M being in addition hydrogen or a salt radical selected from alkali metals, alkaline earth metals, and mono-, di-, or triethanolamine.
the phosphorus ester reactant having a pH of about 5 to 6 before the amine addition and the pH being maintained at pH 7 to 9 after said amine addition at a temperature of at least 80°C and said phosphorus ester reactant being made at a pH of 4 to 5 at a temperature of at least 80°C.

Furtheron, this invention concerns phosphorus-containing surfactant agents having the structure

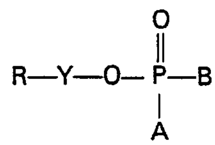

wherein

A is selected from OM, and OYCl,

B is selected from H, OM′, and OYCl with the proviso that M′ is an organic radical,

Y is 2-hydroxypropyl,

R is a primary, secondary or tertiary amine having more than six carbon atoms,

M and M′ are individually an organic radical selected from alkyl or hydroxyalkyl of up to 6 carbon atoms, di-hydroxyalkyl of up to 10 carbon atoms, glyceryl, cycloalkyl of up to 6 carbon atoms, M being in addition hydrogen or a salt radical selected from alkali metals, alkaline earth metals, and mono-, di-, or triethanolamine, obtained by reacting a phosphorus ester reactant of the formula

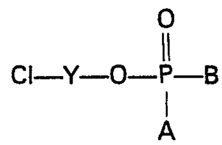

for at least 3 hours with an amine R—H, the phosphorus ester reactant having a pH of about 5 to 6 before the amine addition and the pH being maintained at pH 7 to 9 after said amine addition at a temperature of at least 80°C and said phosphorus ester reactant being made at a pH of 4 to 5 at a temperature of at least 80°C.

The amine reactant can be a primary, secondary, or a tertiary amine. Thus, the amine reactants can carry one, two, or three organic radicals, i.e., they can be primary amines having two hydrogens, secondary amines, or tertiary amines. Preferably, the amine reactant contains not less than 6 or more than 60 carbon atoms total.

The amines employed in the invention contain from one to three organic radicals, i.e., they may contain optionally substituted alkyl, alkenyl, and alkynyl groups, optionally interrupted by hetero atoms, such as oxygen, nitrogen, or sulfur, and can contain other functional groups, e.g., ester or amido moieties, and the amine substituents may, in the case of the secondary and tertiary amines, be themselves linked together to form N-heterocyclic structure, e.g., morpholino. In preferred and more specific embodiments of the invention, the amine reactant is an amidoamine reactant of the formula

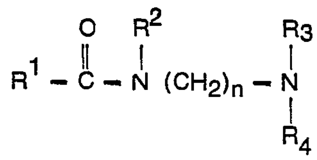 (Ib)

wherein

$R^1$ is alkyl, alkenyl, alkoxy, or hydroxyalkyl of from 5 to 22 carbon atoms each, or aryl or alkyryl of up to 20 carbon atoms.

$R^2$ is hydrogen or alkyl, hydroxyalkyl or alkenyl of up to 6 carbon atoms each or cycloalkyl of up to 6 carbon atoms, preferably of from 2 to 5 carbon atoms, or polyoxyalkalene of up to 10 carbon atoms.

$R^3$ and $R^4$, which may be same or different, are, in the case of the tertiary amines, selected from alkyl, hydroxyalkyl, carboxyalkyl of up to 6 carbon atoms in each alkyl moiety, and polyoxyalkylene of up to 10 carbon atoms; in addition, $R^3$ and $R^4$, taken together with the nitrogen to which they are attached, may represent an N-heterocycle, e.g., a morpholino structure, in which the Y radical is bonded to a ring atom of said N-heterocycle other than the nitrogen of the R moiety; in the case of the secondary amines, one of $R^3$ and $R^4$ is hydrogen, and in the case of the primary amines, both $R^3$ and $R^4$ are hydrogen;

*n* is an integer from 2 to 12.

(The term "polyoxyalkylene radical" as used above in the definition of $R^2$, $R^3$ and $R^4$ may be of the formula $(R^5—O—R^{5'})_m$ wherein $R^5$ and $R^{5'}$ are alkyl of from 1 to 4 carbon atoms and m is an integer from about 2 to 10).

In different specific and preferred embodiment, the secondary and tertiary amine reactants of the invention may be an N-heterocyclic radical which may contain one additional hetero atom (e.g., oxygen or another nitrogen) and contains 5 to 6 total ring carbon atoms; optionally, said heterocyclic radical may be substituted with alkyl and/or hydroxyalkyl of up to 20 carbon atoms each. Typical of such N-heterocyclic radical are imidazolyl, N-alkylmorpholino, alkylpyrimidino, alkoxazolinyl, and the like. The substituents in formula 1(c) are as follows:

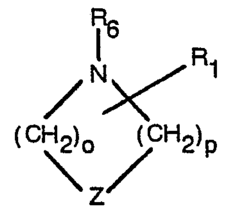

(Ic)

wherein

Z is N or O;

o is an integer from 0 to 3;

P is an integer from 1 to 3 provided that the sum of o + p is from 3 to 4;

$R^1$ is defined as before and is linked to a ring carbon atom; and

$R_6$ is, in the case of the tertiary amines (R), alkyl of from 2 to 6 carbon atoms which may be substituted with a hydroxyl group at the terminal or a non-terminal carbon atom, in the case of the secondary amines (R′), $R_6$ is hydrogen.

The process of the invention is explained below with specific reference to the tertiary, secondary, and primary amine variants.

Tertiary Amine Reactants

When using a tertiary amine reactant for reaction with the phosphate-ester reactant, the process of the invention may be represented by the following generic reaction scheme (1):

(1)

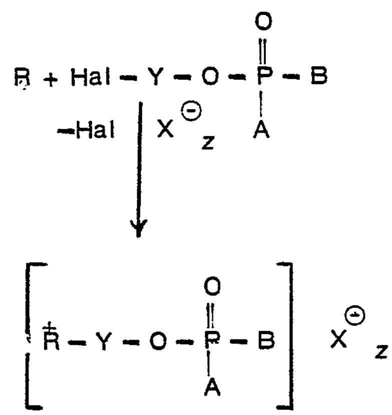


(Ia)

(I)

wherein R is a tertiary amine, i.e., carrying three organic radicals, preferably containing a total of from 6 to 60 carbon atoms:

$X^-$ is an anion; and

*z* is an integer from 0 to 2 and *z* is of a value necessary for charge balance.

When both A and B in formula 1a is —O—Y—Hal, three moles of R are required per mole of phosphate reactant.

When B in formula 1(a) is —O—Y—Hal, two moles of R are required per mole of phosphate ester reactant (Ia).

Still more specific sub-embodiments of reaction scheme (1), i.e., in which particular phosphate ester reactants are employed, can be represented as follows:

(2)

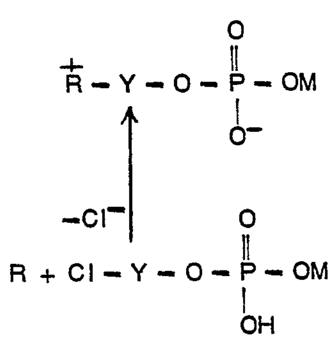


(II)

(IIa)

(3)

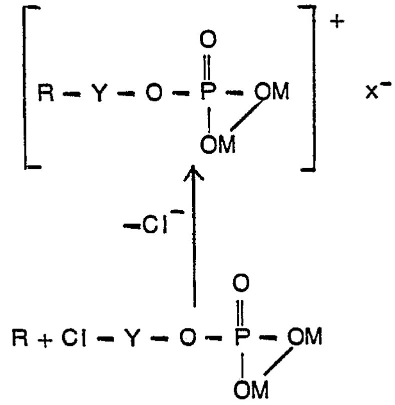


(III)

(IIIa)

(4)

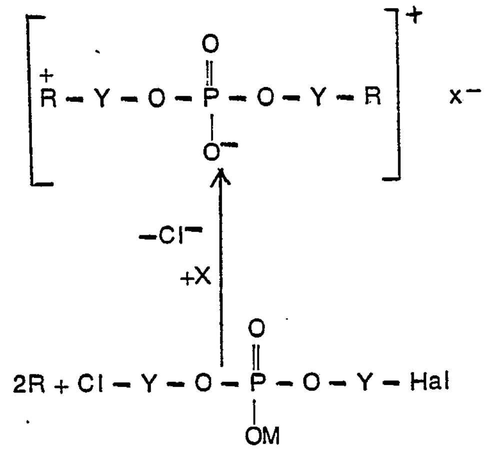


(IV)

(IVa)

wherein the radicals are defined as above.

4a

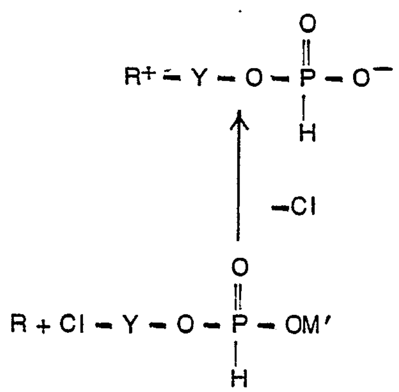


4b

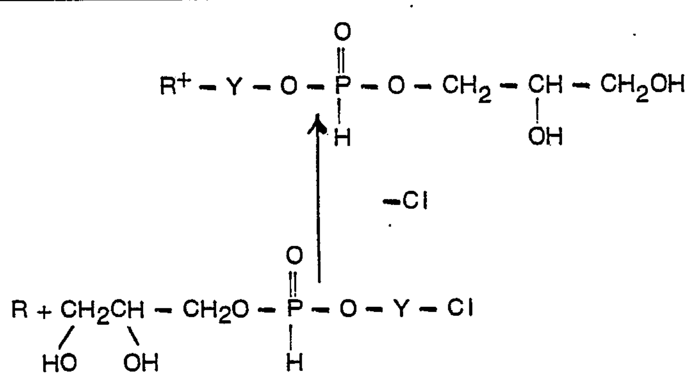

4c

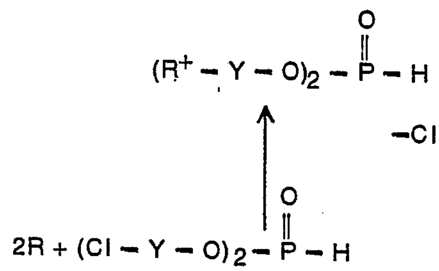


4d

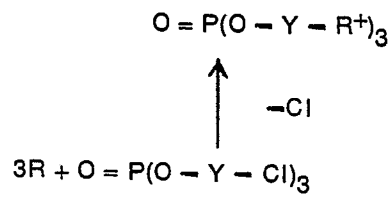


Secondary Amine Reactants

When using a secondary amine reactant with the phosphate-ester reactant, the process of the invention may be represented by the following generic reaction scheme:

(5)

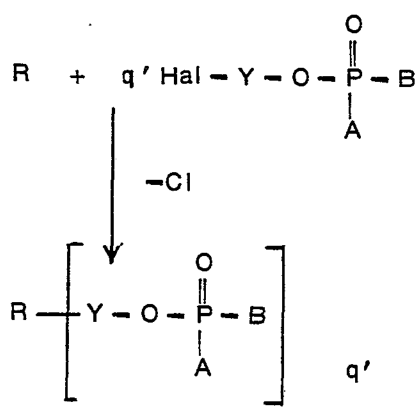


(V)

wherein R is a secondary amine, i.e., carrying two organic radicals, preferably of a total of 6 to 60 carbon atoms, and q′ is an integer from 1 to 2.

It will be understood that when one mole of phosphate ester reactant is employed per mole of secondary amine reactant R (i.e., when $q' = 1$), the reaction proceeds as follows:

(6)

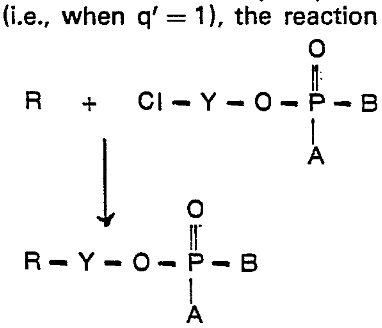

(VI)

'wherein the radicals are defined as above.

When two moles of the secondary amine reactant are supplied (i.e., when $q' = 2$), the reaction leads to a *bis*-compound, as follows:

(7)

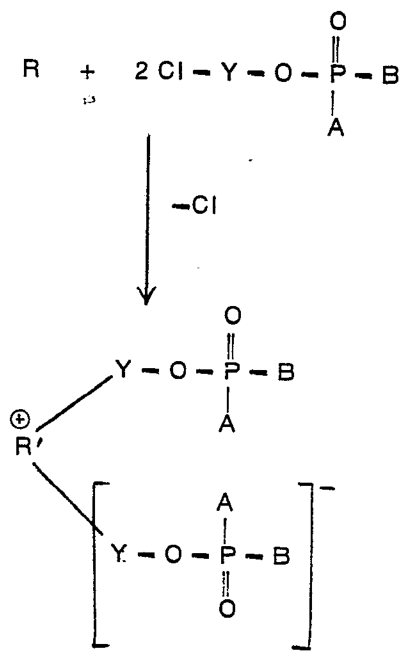


(VII)

wherein the radicals are defined as above, and one of the A or B radicals is $O^-$ as shown, for charge balance.

The secondary amine reactant R can, in preferred and specific aspect, be of the formula I(b) given supra, provided, however, that $R_3$ therein is hydrogen, to give the secondary amine function. Also, R can be a cyclic amine reactant of the formula I(c) given supra, with the proviso that $R_6$ therein is hydrogen, to give the secondary amine function.

Primary Amine Reactants

When using a primary amine reactant with the phosphate ester reactant, the process of the invention may be represented by the following generic reaction scheme:

(8)

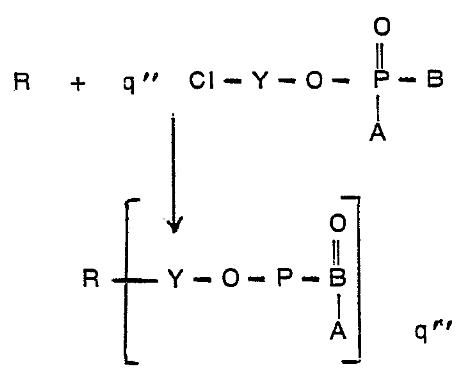

(VIII)

wherein R is a primary amine, i.e., carrying one organic radical, preferably of a total of 6 to 60 carbon atoms, and q'' is an integer from 1 to 3.

It will be understood that when one mole of phosphate ester reactant is employed per mole of primary amine reactant R (i.e., when q'' = 1), the reaction proceeds as follows:

(9)

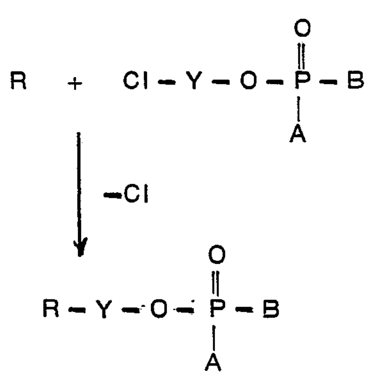


(IX)

wherein the radicals are defined as above.

When two moles of the primary amine reactant are supplied per mol of phosphate ester reactant, i.e., when q″ is 2, the reaction leads to a *bis* compound, as follows:

(10)

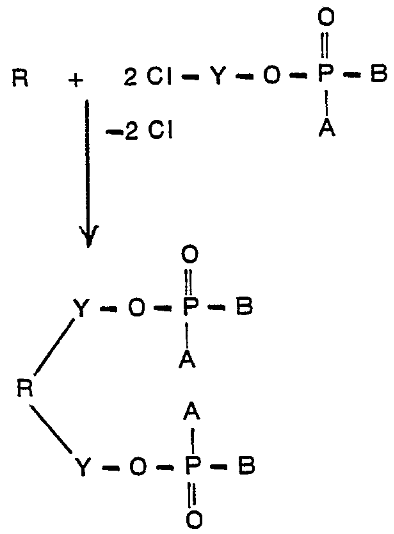


(X)

wherein the radicals are defined as before.

When three moles of the primary amine reactant are supplied, i.e., when q″ is 3, the reaction leads to a *tris*-compound, as follows:

(11)

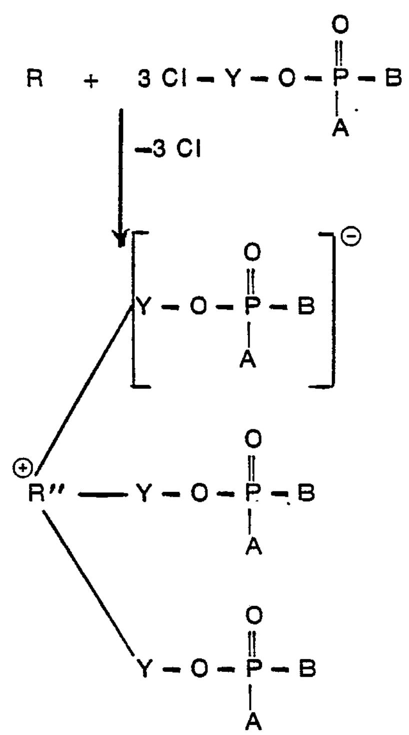


(XI)

wherein the radicals are defined as before, and one of the A or B radicals is $O^-$ as shown, for charge balance.

The primary amine reactant R can, in preferred and specific aspect, be of the formula I(b) given above, provided, however, that $R_3$ and $R_4$ therein are hydrogen, to give the primary amine function.

The reactants required in the processes can be prepared as follows:

Preparation of Intermediate Amine Reactants

The amine reactants R are known or are generally prepared in accordance with conventional techniques. For instance, when making a tertiary amine of the amidoamine type (Ib), this can be prepared by reacting an acid with an aminoalkyl-substituted tertiary (or secondary or primary) amine to result in the amidoamine function.

Alternatively, an acid can be reacted with an aminoalkyl-substituted secondary or primary amine, followed by further treatment of the reaction product with alkylene oxide, to give the tertiary or secondary reactants, respectively. When R is the N-heterocyclic structure (Ic), e.g., imidazolyl, this can be prepared in accordance with known techniques, e.g., as taught in U.S. Patent No. 2,267,965.

Reaction (14) below yields the non-cyclic reactants and Reaction (15) illustrates the preparation of a typical cyclic amine reactant R (Imidazolyl):

14) 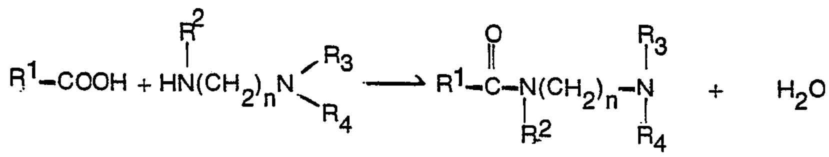

15) 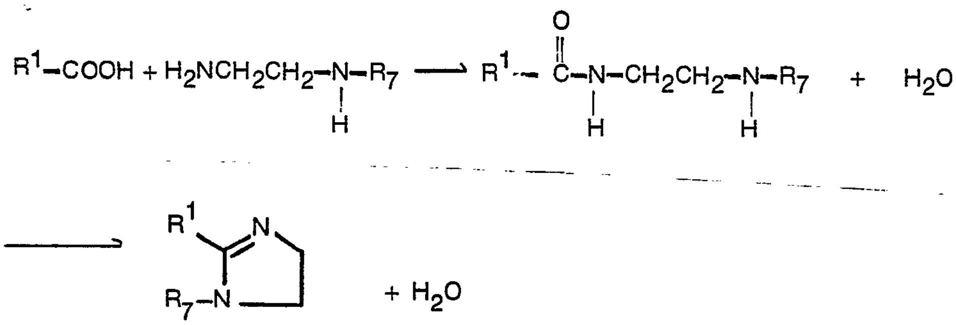

wherein R¹ is defined as above and R⁷ is alkyl of 2 to 6 carbon atoms which may be substituted with a hydroxyl group (at the terminal or a non-terminal carbon atom). This cyclic reactant can be prepared as disclosed in U.S. Patent No. 2,267,965.

A wide variety of commercially available amines are suitable for these reactions. The amine can be primary, secondary or tertiary with only the proviso that the total number of carbons be greater than 6, i.e., to give a hydrophobicity necessary for surface active properties. All other substitutions and modifications of the amine are usable in the present process. Of the commercially available aliphatic amines, there may be mentioned "Armeen" (Armak Chemical), e.g., octyl amine through dodecyl amine. Illustrative aliphatic secondary amines of similar molecular weight include such secondary amines as the "Armeen 2C" (dicocamine) through the disoya amine, tallow amine. Tertiary amines may be simple aliphatic tertiary amines such as the type alkyl dimethyl amine, dialkyl methyl amine, and trialkyl amine, marketed by Armak Chemical Company and an example is "Armeen DM14D" (which is the N-tetradecyl dimethyl amine). Further, ethoxylated aliphatic amines can also be used. These products are marketed under the names of "Ethameen". Any substitution is possible as illustrated elsewhere herein.

Preparation of Phosphate Ester Intermediate Reactants

The preparation of the phosphate ester intermediate reactants I a through VIII a as set forth in the reaction sequences above are also prepared by reactions which are illustrated as follows:

16) 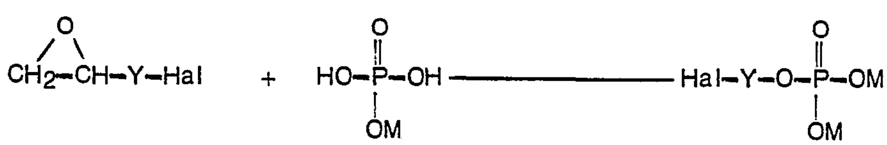 (IIa)

17) 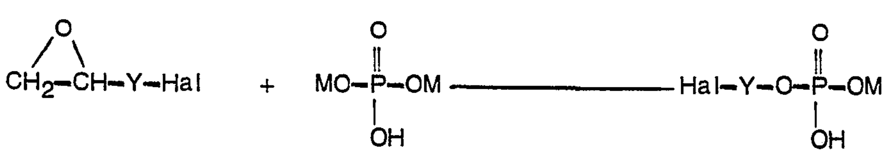 (IIIa)

18) 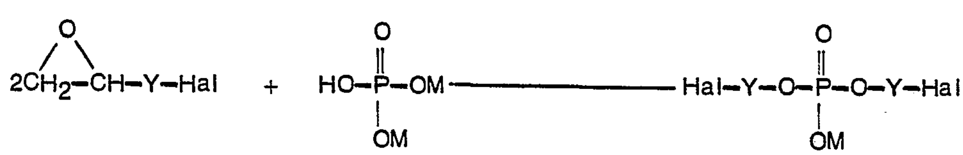 (IVa)

19) 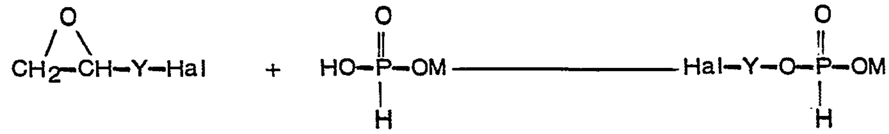 (Va)

20) 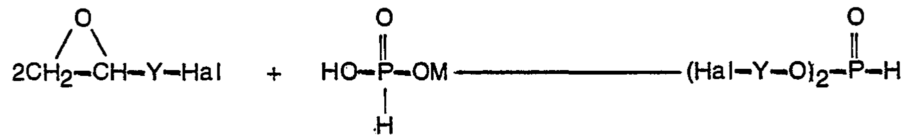 (VIa)

21) 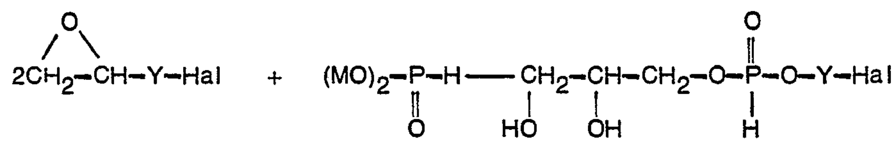 (VIIa)

22) 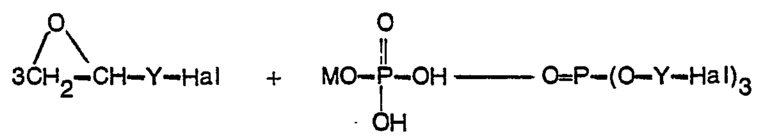 (VIIIa)

The reaction of epichlorohydrin with phosphoric acid and various phosphate salts has not been investigated to an appreciable extent and very little has been written in the literature on this reaction. We have observed that a variety of products can be obtained with variation of certain experimental parameters. The most important of these parameters include the pH of the phosphate salt, the mole ratio of the phosphate salt to the epichlorohydrin and the temperature at which the reaction is run. Reactions No. 16 through 18 occur in an acidic, aqueous solution. Consequently, the reaction proceeds by attack at the epoxide resulting in a 3-chloro-2-hydroxypropyl phosphate intermediate. This intermediate, containing a labile chlorine, is reacted with an amine having at least 6 carbon atoms to give the product conforming to the general phosphobetaine structure. The pH at which this reaction occurs must be strictly controlled. The desired pH range is from 4 to 5. If the pH drops below 4 there is significant hydrolysis of the phosphate ester. If the pH at which the reaction is run is too high, there will be loss of labile organic chlorine. When the reaction is carried out in an alkaline environment of a pH 9.5—10.5 optimally, the resulting intermediate has surprisingly lost its labile organic chlorine and formed a cyclic diester.

The reaction of epichlorohydrin with disodium and trisodium phosphate has been described or reported in the literature; O. Bailly, Compt. Rend. 172, 689—91 (1921); C.A. 15, 1884 (1921) and O. Bailly, Bull. Soc. Chim (4) 31, 848—62 (1922); C.A. 17, 264 (1923). In the reported reaction of disodium hydrogen phosphate with epichlorohydrin a diglycerophosphoric acid diester was thought to have been obtained. As noted above, however we have found that the pH of this system has a profound effect on the product which is obtained. For example, by reaction $NaH_2PO_4$ with epichlorohydrin, the following two products can be obtained, depending on the mole ratio of the two reactants.

If one mole of epichlorohydrin is reacted, the product is 3-chloro-2-hydroxypropyl phosphate (Intermediate Reactant A, below). If two moles are reacted, there is obtained bis(3-chloro-2-hydroxypropyl phosphate); Intermediate Reactant "D", below. These reactions are carried out at a pH of 4—5. Under these conditions, as can be seen from the products obtained, the phosphoric acid moiety attacks the epoxy ring. As the pH is increased to form disodium phosphate, a nucleophilic attack occurs with a displacement of significant quantities of chloride ions. This is shown in the appended drawing, in which

Fig. 1 is a plot showing the effect or pH on chloride generation.

At a pH of 9.5—10.5, theoretical chloride, generation is achieved. Although it was assumed that simultaneous attack was occurring at the epoxy moeity, it was surprisingly discovered that the epoxide group was being retained essentially intact. The product obtained under these conditions was 2,3-epoxy-monosodium propyl phosphate (Intermediate Reactant "C", below). The presence of the epoxy group was subsequently verified by reaction with various amine compounds.

The various intermediates were used to react with various primary, secondary and tertiary amines to form the ultimate amphoteric phosphobetaine compounds. Although similar product would be expected to be obtained when utilizing Intermediate Reactants A and C, distinctly different properties were found for products prepared from these two intermediates. In the case of Intermediate Reactant C, significantly higher foaming products were obtained and this was later shown to be a function of pH of the initial phosphate salt used; this effect is depicted in the drawing wherein

Fig. 2 is a graph captioned "Foam Profile", which is a plot of foam height versus the pH of the initial phosphate salt used.

One of the interesting phenomenon of these products is thus their pH dependent solubility. Not only are the products pH-dependent, but they are also concentration-dependent in that very dilute concentrations tend to become cloudy. The concentration at which this clouding phenomenon occurs is affected by the pH of the initial phosphate salt. This is shown in the appended drawing wherein

Fig. 3 is a plot of pH versus concentration at which cloudiness occurs.

At a pH of 9.5—10.5, there is a leveling off of the solubility.

As noted above, the process parameters must be controlled in order to maintain the reactive organic chlorine atom in the phosphate ester reactants and to avoid hydrolysis of these phosphate esters. Thus, the pH of the phosphate salt employed, the temperature of the reaction and the reaction time were all found to be important in yielding the desired intermediate product. The nature of the intermediate product in turn has a profound effect on the properties of the final products.

Indeed, the surprising reactivity of the labile chlorine containing intermediate reactants, while at the same time their stability against hydrolysis renders eminently suitable for use in the basic process of the invention to yield the ultimate surfactants. When amine reactants are employed, which are sufficiently hydrophobic to surface-active, highly efficacious surface-active products are produced. However, non-surface-active analogues can also be prepared by using low molecular weight or hydrophilic amines.

Specific phosphate ester intermediate reactants which were prepared according to reaction sequences 16—22 *supra* and which were used in the examples, *infra,* in conjunction with certain tertiary amine reactants, are set forth below:

Phosphate Ester Intermediate Reactants

Reactant "A" — Prepared According to Reaction Sequence

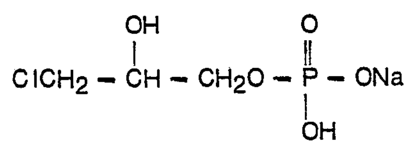

Reactant "B" — Prepared According to Reaction Sequence

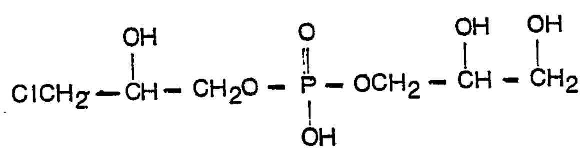

Reactant "C" — Prepared According to Reaction Sequence

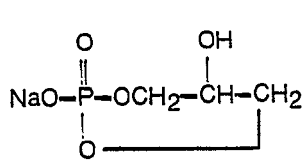

and/or

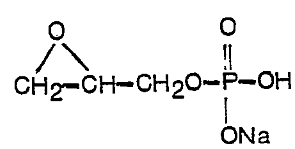

Reactant "D" — Prepared According to Reaction Sequence

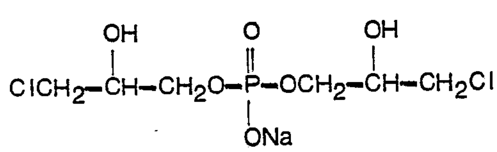

Reactant "E" — Prepared According to Reaction Sequence

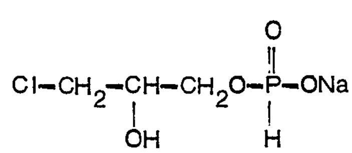

Reactant "F" — Prepared According to the Reaction Sequence

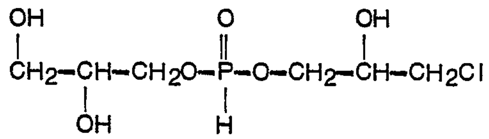

Reactant "G" — Prepared According to Reaction Sequence

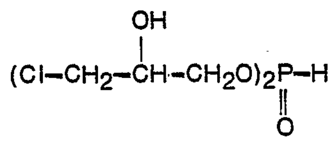

Reactant "H" — Prepared According to Reaction Sequence

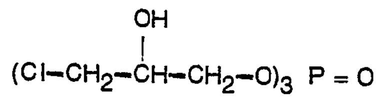

In carrying out the reactions 1 to 13 as set forth above leading to the ultimate compounds of the invention, the amine intermediate reactant R is reacted with the appropriate phosphate ester intermediate reactant and these reactions are generally carried out in an aqueous system at 80—100°C.

The optimum concentration range of the reactants in reactions 1 to 13 in aqueous solution is from 30—50% and most preferably the reactants constitute about 40% of the aqueous reactant mixture.

The phosphate ester reactant should have a pH of about 5 to 6 before the amine addition and the pH should be maintained at between 7 to 9 after said amine addition; below a pH of 7 the rate of quaternization slows significantly and, of course, the pH of the amines used themselves limit the pH on the alkaline topside at about pH 9.

As noted above, the reactions 1 to 13 are generally carried out at a temperature of about 80—100°C. The reaction is preferably initially conducted at a temperature of 80—100°C and may be conducted under some pressure, e.g., under 1,33 bar of nitrogen. However, under pressure, higher temperatures can be used, e.g., temperatures up to about 130°C and the ceiling temperature is determined by the sensitivity of the reactants, e.g., the amine reactant, since it will be found that color loss and evidence of other degradation occurs at unduly high temperatures.

Many specific examples of ultimate phosphobetaine preparation are set forth below.

The ultimate reaction products are good surfactants and quite unexpectedly exhibit good foam volume and superior foam stability in comparison to commercially available amphoteric and zwitterionic surfactants. This was determined by an adaptation of the well known Ross-Miles foam test principle ["Oil and Soap] 18, 99—102 (1941)] in the following manner.

Lanolin, anhydrous, cosmetic grade is mixed with dioxane (technical grade) in the proportion of 2.5 grams lanolin and 100 grams of dioxane. The lanolin is first mixed with 25 cc of dioxane. This mixture is heated over a steam bath to 45°C in order to dissolve the lanolin in the dioxane. The remainder of the dioxane is then added and mixed. This lanolin-dioxane solution, which is stored in an amber bottle, should be prepared fresh on the day that the tests are run.

The composition to be tested is diluted by adding 376 cc of distilled water to 4 grams of the composition and then by adding 20 cc of the lanolin-dioxane solution described above while mixing. Heat is produced when the lanolin-dioxane solution is added to the solution of the composition in water and care must be taken in adjusting the temperature of this solution to 24—25°C. Both of these intermediate solutions should therefore be adjusted to 23°C before mixing. The cooling of the lanolin-dioxane solution should be gradual in order to avoid precipitation of the lanolin. This will produce a final solution with a temperature of 24—25°C.

The final solution of the composition to be tested, water, dioxane and lanolin described above, is then run in a modified Ross-Miles foam column in the usual way. All tests are conducted in duplicate, and the average of the two results is taken. Foam stability is determined by measuring the decay in foam height after two minutes, expressed as a percentage of the original height.

Typical foam values obtained utilizing the above procedure for an alkylamidophosphobetaine, an alkylamido betaine and an alkylamido sultaine are listed below:

| | Example No. | Foam Volume (ml) | % Decay After 2 min. |
|---|---|---|---|
| Lauric Myristic Amido 3-Hydroxypropyl Phosphobetaine | 10 | 250 | 4.0 |
| Cocamido Disodium 3-Hydroxypropyl Phosphobetaine | 9 | 240 | 10 |
| Cocamido Propylbetaine | — | 225 | 31.0 |
| Cocamido Propylsultaine | — | 200 | 60.0 |

As can be seen from the above results, the phosphobetaines made by the process of the invention exhibit excellent foam volume and stability, whereas the stability of the betaines and sultaines is significantly less.

In another series of tests, additional species of the phosphobetaine compounds of the invention were tested by a "cylinder shake test" for the evaluation of foaming characteristics.

In this test, test solutions containing 0.1% by weight of the candidate surfactant in water of 100 ppm hardness (calcium to magnesium ratio 3:2) were used and placed in 100 ml stoppered cylinders which had been cleaned so that water drains down its walls in an unbroken film. Each cylinder filled with test solution was shaken 20 times in a standard manner and net foam in ml is noted one minute and again five minutes after shaking. The tests were run in three replicates. The results were as follows:

| Lauric/Myristic Type | Example Number | 1 Minute | 5 Minutes |
|---|---|---|---|
| Lauric Myristic Amido Betaine | — | 67 | 60 |
| Sodium Lauryl Sulfate | — | 85 | 74 |
| Lauric Myristic Amido Glyceryl Phosphobetaine | 10 | 86 | 78 |

| Coco Type | Example Number | 1 Minute | 5 Minutes |
|---|---|---|---|
| Cocobetaine | — | 65 | 56 |
| Cocamidobetaine | — | 70 | 63 |
| Cocamido Glyceryl Phosphobetaine | 21 | 71 | 74 |

In addition, the compounds of the present invention possess a surprisingly low ocular irritation potential when compared to commercially available amphoteric and zwitterionic surfactants. The test employed is the modified Draize Test (J.H. Draize et al, Toilet Goods Assn. No. 17, May, 1852, No. 1 Proc. Sci. Sect.).

In this method, a 0.1 ml sample of a neutral solution of the compound under investigation is dropped into one eye of an albino rabbit, the other eye serving as a control. Six rabbits are employed for each compound.

Observations are made after 1, 24, 48, 72 and 96 hours and 7 days after initial instillation; second and third instillations are made after the 24 and 48 hour readings. Results may vary from substantially no change, or only a slight irritation in the appearance of the rabbit's eye after 7 days, to severe irritation and/or complete corneal opacity. Ocular lesions are scored for the cornea, iris and conjuctiva with a higher number indicating greater ocular irritation and the scores are added to give a total numerical value for each reading for 6 rabbits and average. The averaged score is an indication of the irritation potential of the composition under test. Based on the averaged score, descriptive irritation evaluation may be given, e.g., none, slight, moderate, severe, etc.

Typical results for a betaine, sultaine and a phosphobetaine in accordance with the present invention when subjected to the above test procedure are as follows:

| | Eye Irritation Potential | | | | | | Irritant |
|---|---|---|---|---|---|---|---|
| Compound | 1 hr. | 24 hr. | 48 hr. | 72 hr. | 96 hr. | Day 7 | Rating |
| Lauric Myristic Amido 3-Hydroxypropyl Phosphobetaine (Example 10) | 7.7 | 0.3 | 1.3 | 1.0 | 0.0 | — | slight |
| Cocamido Propylbetaine | 11.7 | 4.2 | 9.3 | 13.2 | 11.2 | 5.8 | severe |
| Cocamido Propylsultaine | 15.0 | 8.5 | 15.6 | 25.0 | — | — | severe |

All tests were conducted at a concentration of 3% wt/wt.

In further series of tests carried out as above, but using only one test rabbit per compound, the following results were obtained:

| | Example | | | Day | | | Irritant |
|---|---|---|---|---|---|---|---|
| | Number | 1 | 2 | 3 | 4 | 7 | Rating |
| Lauric Myristic Disodium Amido Hydroxypropyl Phosphobetaine | 14 | 7 | 0 | 1 | 1 | 0 | very slight |
| Comparison | | | | | | | |
| Lauric Myristic Amido Betaine | — | 8 | 15 | 25 | — | — | severe |
| Cocobetaine | — | 23 | 26 | 19 | 17 | 25 | severe |
| Cocamidobetaine | — | 21 | 19 | 14 | 9 | 6 | severe |
| Sodium Lauryl Sulfate | — | 18 | 16 | 16 | 16 | 10 | severe |

As can be readily seen, the phosphobetaines in accordance with the present invention exhibit only slight ocular irritation, whereas the betaines and sultaines are severe irritants.

The following examples illustrate the processes of the invention and the novel intermediates utilized therein.

Example 1

REACTANT "A"

60.00 parts of deionized water are charged into a suitable reactor to which 22.58 parts of $NaH_2PO_4$ and 0.70 parts of NaOH are charged under good agitation. Mix well until a solution is obtained. 17.42 parts of Epichlorohydrin is charged under good agitation. The reactor is sealed and 1,33 bar $N_2$ is applied. Heat to 80—85°C. holding the heated mixture at this temperature for 2 hours after the batch clears (approximately 3 hours total). Reaction is complete when theoretical reduction in acid value is obtained. Inorganic chloride will be less than 0.50%.

The product is an aqueous solution of a novel product having the following structure:

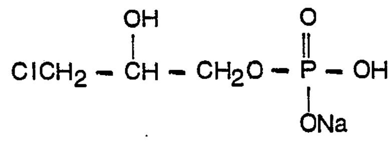

## Example 2

REACTANT "B"

60.00 parts of deionized water are charged into a suitable reactor to which 17.37 parts of $Na_2HPO_4$ are charged. Mix well until a solution is obtained. 22.63 parts of Epichlorohydrin are charged under good agitation. The reactor is sealed and 1,33 bar $N_2$ is applied. Heat to 80—85°C. holding the heated mixture at this temperature for approximately 2 hours after the batch clears (approximately 3 hours total). Reaction is complete when theoretical chloride is obtained and theoretical reduction in acid value is realized.

The product is an aqueous solution of a novel product having the following structure:

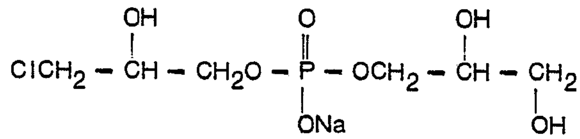

## Example 3

REACTANT "C" (Comparison

60.00 parts of deionized water are charged into a suitable reactor to which 24.22 parts of $Na_2HPO_4$ are charged. Mix well until a solution is obtained. 15.78 parts of Epichlorohydrin are charged under good agitation. The reactor is sealed and 1,33 bar $N_2$ is applied. Heat to 80—85°C. holding the heated mixture at this temperature for 2 hours after the batch clears (approximately 3 hours total). Reaction is complete when theoretical reduction in acid value and theoretical inorganic chloride values are obtained.

The product is an aqueous solution of a novel product having the following structure:

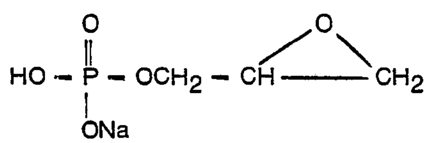 and 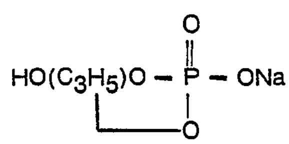

## Example 4

REACTANT "D"

50.00 parts of deionized water are charged into a suitable reactor to which 17.09 parts of $NaH_2PO_4$ and 0.70 parts of NaOH are charged. Mix well until a solution is achieved. 22.91 parts of Epichlorohydrin is charged under good agitation. Reactor is sealed and 1,33 bar $N_2$ is applied. Heat is applied to approximately 80—85°C and the heated mixture held at this temperature for approximately 3 hours after the batch clears (approximately 5 hours total). Reaction is complete when acid value is reduced by the theoretical amount. Inorganic chloride will be less than 0.5%.

The product is an aqueous solution of a novel product having the following structure:

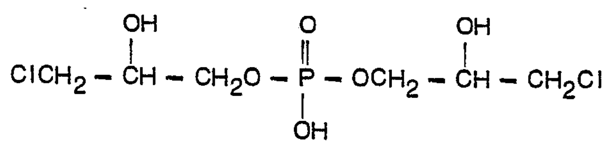

## Example 5

REACTANT "E"

60.00 parts of deionized water are charged into a suitable reactor to which 21.17 parts of $NaH_2PO_3$ is added. Mix well until solution is achieved. 18.83 parts of Epichlorohydrin is charged under good agitation. Reactor is sealed and 1,33 bar $N_2$ is applied. Heat is applied to 80—85°C, and the reaction mixture is held at this temperature for approximately 3 hours after the batch clears (aprox 5 hours total). Reaction is complete when acid value is reduced by theoretical amount.

The amount in an aqueous solution of a novel product having the following structure:

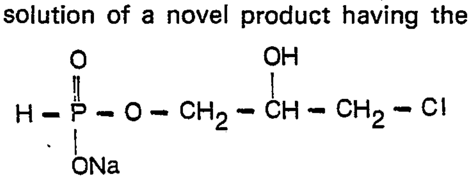

Example 6

REACTANT "F"

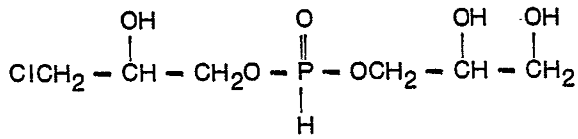

To 60.00 parts of soft water in a suitable reactor, slowly charge 16.20 parts of $Na_2HPO_3$ under good agitation. Heat to 40—50°C. Slowly charge 23.79 parts epichlorohydrin under good agitation. Seal reactor and apply 1,33 bar nitrogen. Heat to 90—95°C and hold 3—4 hours. Reaction is complete when theoretical reduction in acid value has occurred and inorganic chloride reaches 50% of theoretical.

Example 7

REACTANT "G"

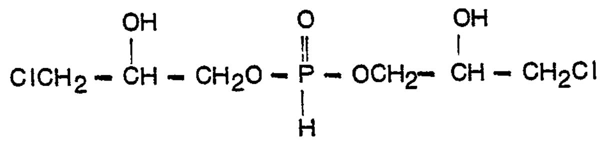

To 60.00 parts of soft water in a suitable reactor, slowly charge 12.28 parts $NaH_2PO_3$ under good agitation. Heat slowly to 40—45°C. Slowly charge 27.72 parts epichlorohydrin under good agitation. Seal reactor and apply 1,33 bar nitrogen. Heat to 90—95°C and hold 3—4 hours. Reaction is complete when there is no acid value.

Example 8

REACTANT "H"

To 60.00 parts of water charge 21.1 parts of mono sodium phosphate in a suitable reaction vessel under good agitation. Apply heat to 50—55°C. Slowly charge 27.9 parts epichlorohydrin. Seal reactor and apply 1,33 bar $N_2$. Heat slowly to 80—85°C and hold 2 to 3 hours. Reaction is complete when acid value has been reduced to vanishingly small levels. Inorganic chloride levels will likewise be vanishingly small.

The product is an aqueous solution of a novel product having the following structure:

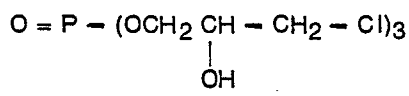

Example 9

20.5 parts of Reactant "A" and 60.0 parts of water are charged in a reacting vessel under good agitation. Heat is applied to 50°C. 19.5 parts of molten 3-cocamidopropyl dimethylamine are slowly charged under good agitation. Heating is continued to 90—95°C and held at this temperature for 3 to 4 hours. Reaction reached 97% during this time via inorganic chloride and tertiary amine analysis.

The product is an aqueous solution of a novel product having the following structure:

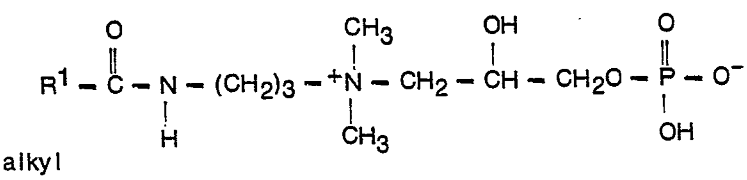

$R^1 = C_7$ to $C_{17}$ alkyl

Example 10

20.8 parts of Reactant "A" and 60.0 parts of water are charged in a reacting vessel under good agitation. Heat is applied to approximately 50°C. 19.2 parts of a 70/30 blend of lauramidopropyl dimethylamine and myristamidopropyl dimethylamine are slowly charged to the above solution using good agitation. Heating is continued to 90—95°C and held at this temperature for 3 to 4 hours. Reaction reached 97% during this time via inorganic chloride and tertiary amine analysis.

The product is an aqueous solution of a novel product having the following structure:

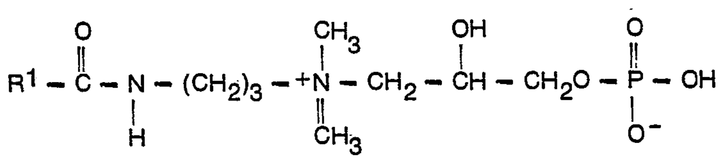

$R_1$ = 70% $C_{11}$ alkyl

30% $C_{13}$ alkyl

Example 11

22.88 parts of Reactant "A" and 60.0 parts water are charged into a suitable reaction vessel under good agitation. Heat is applied to 50°C. 17.12 parts of 1-hydroxyethyl-2-alkyl-2-imidazoline (the alkyl has 7 to 17 carbon atoms) are charged under good agitation. Heating is continued to 90—95°C. and the heated mixture held at this temperature for 4 hours. Reaction reaches 97% during this time via inorganic chloride and tertiary amine analysis.

The product is an aqueous solution of a novel product having the following structure:

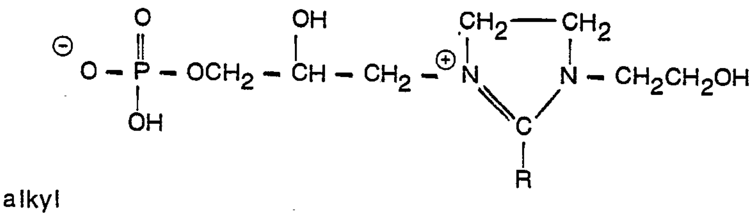

R = $C_7$ to $C_{17}$ alkyl

Example 12

20.8 parts of Reactant "A" and 60.0 parts of water are charged into a suitable reaction vessel under good agitation. Heat is applied to 50°C. 19.2 parts of 1-hydroxyethyl 2-alkyl-2-imidazoline (the alkyl being $C_{17}$) are charged under good agitation. Heating is continued to 90—95°C. and the heated mixture held at this temperature for 4 hours. Reaction reaches 97% during this time via inorganic chloride and tertiary amine analysis.

The product is an aqueous solution of a novel product having the following structure:

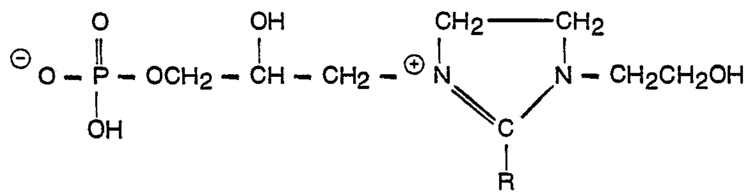

R = $C_{17}$ alkyl

Example 13

26.8 parts of Reactant "B" and 60.0 parts of water are charged in a reacting vessel under good agitation. Heat is applied to approximately 50°C. 13.2 parts of 3-cocamidopropyl dimethylamine are slowly charged to the above solution using good agitation. Heating is continued to 90—95°C and the heated mixture held at this temperature for 3 to 4 hours. Reaction reaches 98% during this time via inorganic chloride and tertiary amine analysis.

The product is an aqueous solution of a novel product having the following structure:

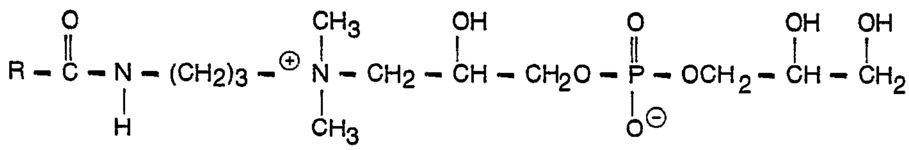

R = $C_7 - C_{17}$ alkyl

Example 14

26.4 parts of Reactant "B" and 60.0 parts of water are charged in a reacting vessel under good agitation. Heat is applied to approximately 50°C. 13.6 parts of a 70/30 blend of 3-lauramidopropyl diethylamine and 3-myristamidopropyl diethylamine are slowly charged to the above solution using good agitation. Heating is continued to 90—95°C and the heated mixture held at this temperature for 3 to 4 hours. Reaction reaches 98% during this time via inorganic chloride and tertiary amine analysis.

Example 15

14.4 parts of Reactant "D" and 60.0 parts of water are charged in a reacting vessel under good agitation. Heat is applied to 50°C. 25.6 parts of molten 3-cocamidopropyl dimethylamine are slowly charged under good agitation. Heating is continued to 90—95°C. and the heated mixture held at this temperature for 3 to 4 hours. Reaction reaches 98% during this time via inorganic chloride and tertiary amine analysis.

The product is an aqueous solution of a novel product having the following structure:

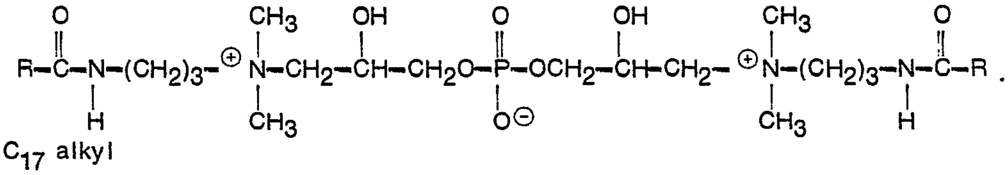

R = $C_7 - C_{17}$ alkyl

Example 16

15.3 parts of Reactant "D" and 60.0 parts of water are charged in a reacting vessel under good agitation. Heat is applied to approximately 50°C. 24.7 parts of 70/30 blend of 3-lauramidopropyl dimethylamine and 3-myristamidopropyl dimethylamine are slowly charged to the above solution using good agitation. Heating is continued to 90—95°C and the heating mixture held at this temperature for 3 to 4 hours. Reaction reaches 98% during this time via inorganic chloride and tertiary amine analysis.

The product is an aqueous solution of a novel product having the following structure:

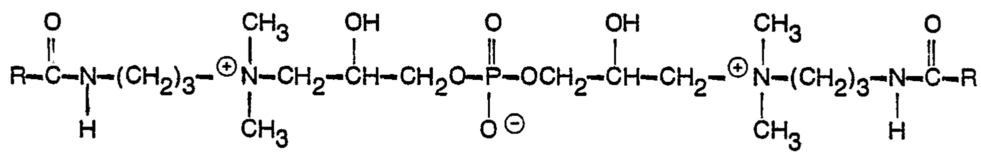

R = 70% $C_{11}$ alkyl

30% $C_{13}$ alkyl

Example 17

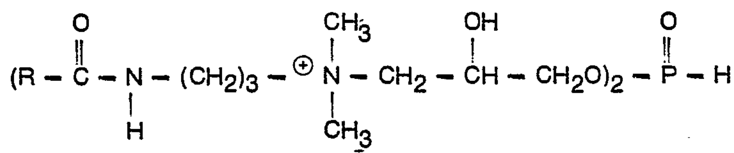

To 60.00 parts of soft water in a suitable reactor, charge 19.66 parts of Reactant "G" under good agitation. Heat to 45—50°C and charge 20.34 parts of 3 cocamido propyl dimethyl amine under good agitation. Heat to 90—95°C and hold 4—5 hours. Reaction is complete when theoretical inorganic chloride is generated and when residuea tertiary nitrogen levels become vanishingly small.

The product is an aqueous solution of the above material. $R = C_7 - C_{17}$

Example 18

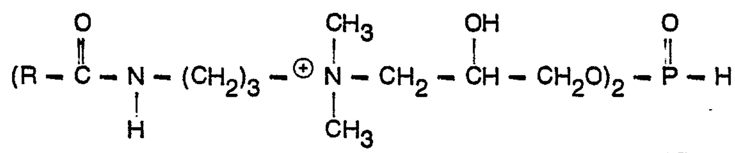

To 60.00 parts of soft water in a suitable reactor, charge 20.81 parts of Reactant "G" under good agitation. Heat to 5—50°C and charge 19.19 parts of 3 lauramido propyl dimethyl amine under good agitation. Heat to 90—95°C and hold 4—5 hours. Reaction is complete when theoretical inorganic chloride is generated and when residual tertiary nitrogen levels become vanishingly small.

The product is an aqueous solution of the above material. $R = C_{11}$

Example 19

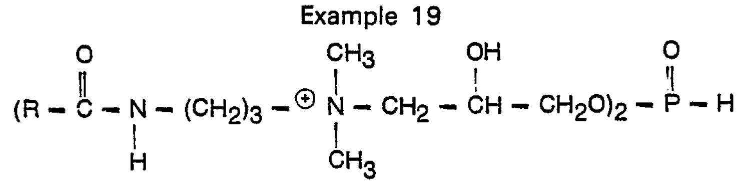

To 60.00 parts of soft water in a suitable reactor, charge 23.10 parts of Reactant "G" under good agitation. Heat to 45—50°C and charge 16.90 parts of 3 caprylamido propyl dimethyl amine under good agitation. Heat to 90—95°C and hold 4—5 hours. Reaction is complete when theoretical inorganic chloride is generated and when residual tertiary nitrogen levels become vanishingly small.

The product is an aqueous solution of the above material $R = C_7$

Example 20

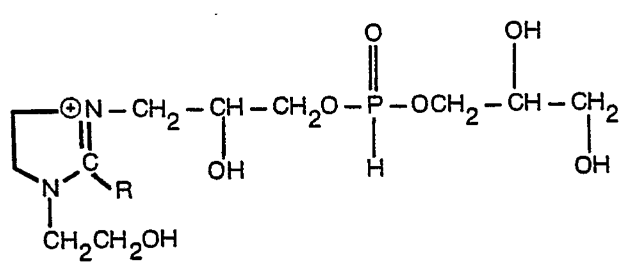

To 60.00 parts of soft water in a suitable reactor, charge 22.14 parts of Reactant "F" under good agitation. Heat to 45—50°C and charge 17.86 parts of 1 hydroxyethyl 2 alkyl 2 imidazoline (alkyl being $C_7$ to $C_{17}$) under good agitation. Heat to 90—95°C and hold 4—5 hours. Reaction is complete when theoretical inorganic chloride is generated and when residual tertiary nitrogen levels become vanishingly small. The product is an aqueous solution of the above material. $R = C_7 - C_{17}$

Example 21

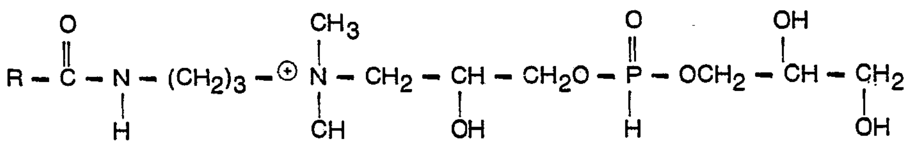

To 60.00 parts of soft water in a suitable reactor, charge 21.12 parts of Reactant "F" under good agitation. Heat to 45—50°C and charge 18.88 parts parts of 3 cocamido propyl dimethyl amine under good agitation. Heat to 90—95°C and hold 4—5 hours. Reaction is complete when theoretical inorganic chloride is generated and when residual tertiary nitrogen levels become vanishingly small.

The product is an aqueous solution of the above material $R = C_7—C_{17}$

Example 22

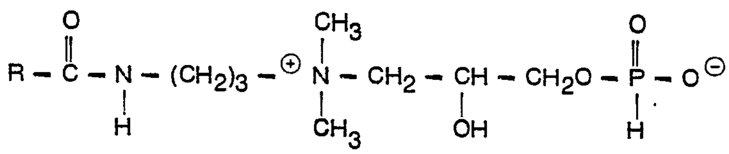

To 60.00 parts of soft water in a suitable reactor, charge 20.06 parts of Reactant "E" under good agitation. Heat to 40—45°C and charge 19.94 parts of a 70/30 blend of 3 lauramido propyl dimethyl amine + 3 myristamido propyl dimethyl amine under good agitation. Heat to 90—95°C and hold 4—5 hours. Reaction is complete when theoretical inorganic chloride is generated and when residual tertiary nitrogen levels become vanishingly small.

The product is an aqueous solution of the above material $R = 70\%—C_{11}/30\%—C_{13}$.

Example 23

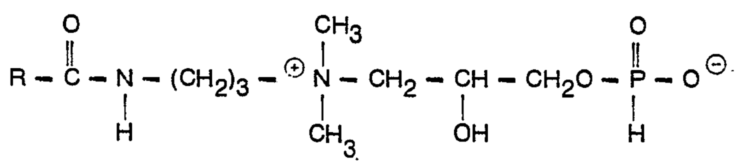

To 60.00 parts of soft water in a suitable reactor, charge 20.82 parts of Reactant "E" under good agitation. Heat to 45—50°C and charge 19.18 parts of 3 lauramido propyl dimethyl amine under good agitation. Heat to 90—95°C and hold 4—5 hours. Reaction is complete when theoretical inorganic chloride is generated and when residual tertiary nitrogen levels become vanishingly small.

The product is an aqueous solution of the above material. $R = C_{11}$

Example 24

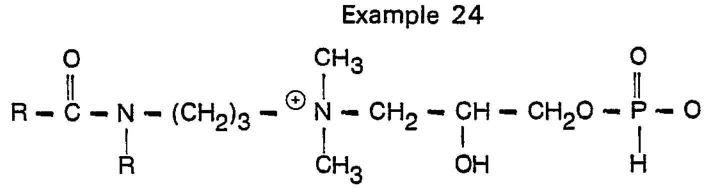

To 60.00 parts of soft water in a suitable reactor, charge 23.10 parts of Reactant "E" under good agitation. Heat to 45—50°C and charge 16.9 parts of 3 caprylamido propyl dimethyl amine under good agitation. Heat to 90—95°C and hold 4—5 hours. Reaction is complete when theoretical inorganic chloride is generated and when residual tertiary nitrogen levels become vanishingly small.

The product is an aqueous solution of the above material $R = C_7$

Example 25

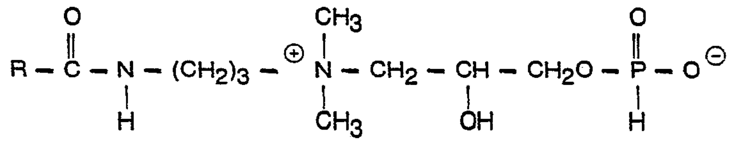

To 60.00 parts of soft water in a suitable reactor, charge 24.45 parts of Reactant "E" under good agitation. Heat to 45—50°C and charge 15.55 parts of 3 caprylamido propyl dimethyl amine under good agitation. Heat to 90—95°C and hold 4—5 hours. Reaction is complete when theoretical inorganic chloride is generated and when residual tertiary nitrogen levels become vanishingly small. The product is an aqueous solution of the above material $R = C_5$.

Example 26

To 60.00 parts of soft water in a suitable reactor, charge 12.01 parts of Reactant "H" under good agitation. Heat to 45—50°C and slowly charge 27.99 parts of cocamidopropyl dimethyl amine under good agitation. Heat to 90—95°C and hold for 4—5 hours. Reaction is complete when inorganic chloride reaches theoretical and residual tertiary nitrogen levels are vanishingly small.

The product is an aqueous solution of the material shown below:

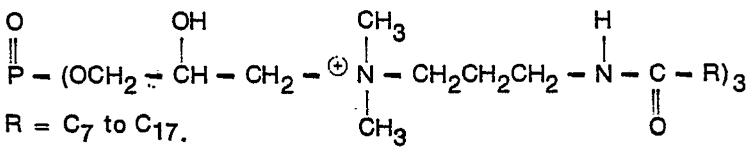

$R = C_7$ to $C_{17}$.

Example 27

To 60.00 parts of soft water in a suitable reactor, charge 12.17 parts of Reactant "H" under good agitation. Heat to 45—50°C and slowly charge 27.83 parts of 1-hydroxyethyl 2-alkyl imidazoline (alkyl being $C_7$ to $C_{17}$) under good agitation. Heat to 90—95°C and hold for 4—5 hours. Reaction is complete when inorganic chloride reaches theoretical and residual tertiary nitrogen levels are vanishingly small.

The product is an aqueous solution of the material shown below:

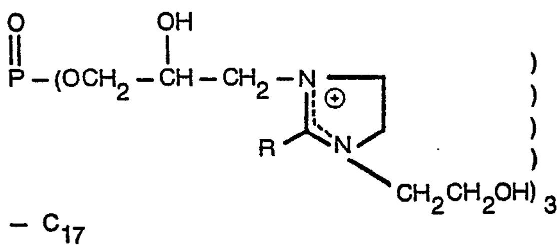

$R = C_7 - C_{17}$

**Claims**

1. A process for the production of phosphorus-containing surface active agents, which process comprises reacting a phosphorus ester reactant selected from phosphate di- and tri-esters and phosphite di-esters, having a labile chlorine with an amine, characterized by the reaction of an amine having at least 6 carbon atoms for at least 3 hours with a phosphorus ester reactant of the formula:

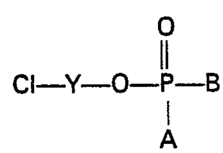

wherein

A is selected from OM, and OYCl,

B is selected from H, OM' and OYCl with the proviso that M' is an organic radical,

Y is 2-hydroxypropyl,

M and M' are individually an organic radical selected from alkyl or hydroxyalkyl of up to 6 carbon atoms, dihydroxyalkyl of up to 10 carbon atoms, glyceryl, cycloalkyl of up to 6 carbon atoms, M being in addition hydrogen or a salt radical selected from alkali metals, alkaline earth metals, and mono-, di-, or triethanolamine,

the phosphorus ester reactant having a pH of about 5 to 6 before the amine addition and the pH being maintained at pH 7 to 9 after said amine addition at a temperature of at least 80°C and said phosphorus ester reactant being made at a pH of 4 to 5 at a temperature of at least 80°C.

2. Process as claimed claim 1 wherein said phosphorus ester is of the formula:

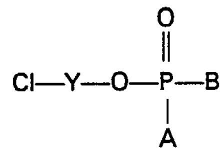

wherein
A is OM
B is OM′
M is Na
M′ is Glyceryl

3. Process as claimed in claim 1 wherein said phosphorus ester reactant is of the formula:

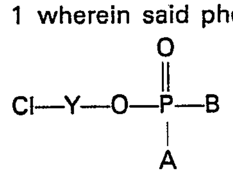

wherein
A is OM
B is OM′
M is Glyceryl
M′ is 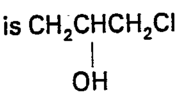

4. Process as claimed in claim 1 wherein said phosphorus ester reactant is of the formula:

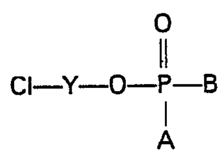

wherein;
A is OM
B is H
M⊕ is Glyceryl

5. Process as claimed in claim 1 wherein said phosphorus ester reactant is of the formula:

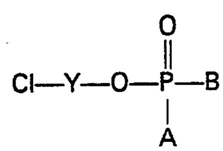

wherein
A is OM
B is H
M is 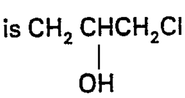

6. Process as claimed in claim 1 wherein said phosphorus ester reactant is of the formula:

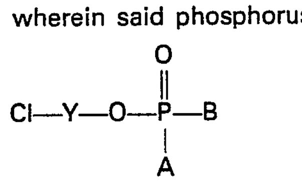

wherein

Both A and B are 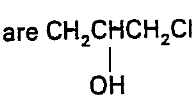

7. Process as claimed in claim 1 wherein said phosphorus ester reactant is of the formula:

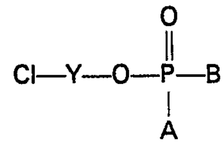

wherein
A is OM
B is OYCl
M is Na

8. Phosphorus-containing surfactant agent having the structure

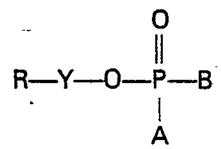

wherein
A is selected from OM, and OYCl,
B is selected from H, OM', and OYCl with the proviso that M' is an organic radical,
Y is 2-hydroxypropyl,
R is a primary, secondary or tertiary amine having more than six carbon atoms,
M and M' are individually an organic radical selected from alkyl or hydroxyalkyl of up to 6 carbon atoms, di-hydroxyalkyl of up to 10 carbon atoms, glyceryl, cycloalkyl of up to 6 carbon atoms, M being in addition hydrogen or a salt radical selected from alkali metals, alkaline earth metals, and mono-, di- or triethanolamine, obtained by reacting a phosphorus ester reactant of the formula

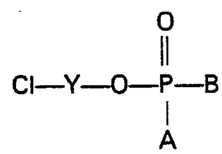

for at least 3 hours with an amine R—H, the phosphorus ester reactant having a pH of about 5 to 6 before the amine addition and the pH being maintained at pH 7 to 9 after said amine addition at a temperature of at least 80°C and said phosphorus ester reactant being made at a pH of 4 to 5 at a temperature of at least 80°C.

9. Phosphorus-containing surfactant as claimed in claim 8 having the structure:

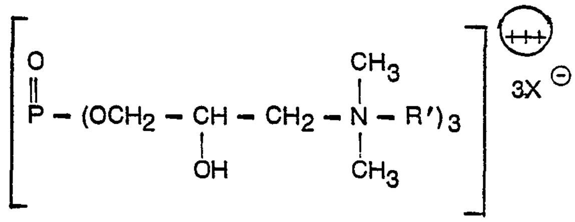

wherein
R' is a tertiary amine radical of from 6 to 40 carbon atoms; and
X is an anion.

10. Phosphorus-containing surfactant as claimed in claim 9 having the structure.

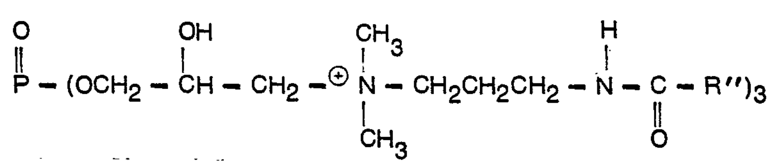

and wherein R'' is alkyl with 7 to 17 carbon atoms.

11. Phosphorus-containing surfactant as claimed in claim 9 having the structure

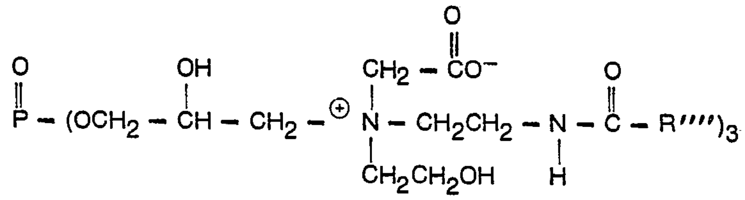

and wherein 70% of $R''''$ is alkyl with 11 carbon atoms.

12. Phosphorus-containing surfactant of the formula

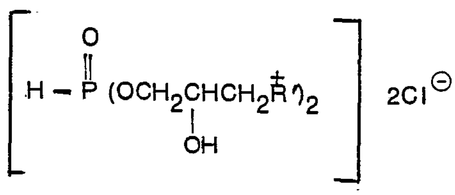

wherein $R'$ is a quaternary ammonium radical.

13. Phosphorus-containing surfactant as claimed in claim 12 wherein $R'$ is

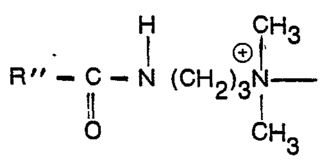

and $R''$ is $C_7$ to $C_{17}$ alkyl.

14. Phosphorus-containing surfactant as claimed in claim 12 wherein $R'$ is

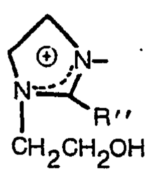

and $R''$ is $C_7$ to $C_{17}$ alkyl.

15. Phosphorus-containing surfactant of the formula

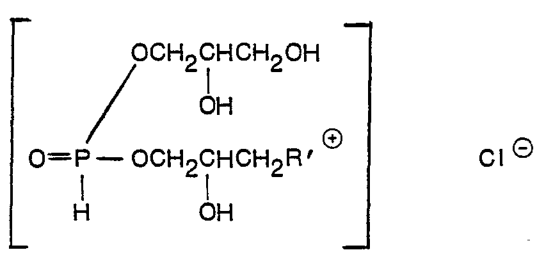

wherein $R'$ is a quaternary ammonium radical.

16. Phosphorus-containing surfactant as claimed in claim 15 wherein: $R'$ is

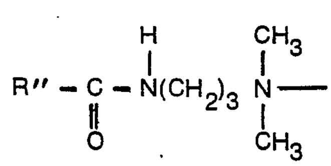

and $R''$ is $C_7$ to $C_{17}$ alkyl.

17. Phosphorus-containing surfactant as claimed in claim 15 wherein:
R′ is

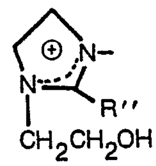

R″ is $C_7$ to $C_{17}$ alkyl.

**Revendications**

1. Procédé pour la fabrication d'agents tensioactifs contenant du phosphore, procédé consistant à faire réagir un réactif du type ester phosphorique choisi parmi les diesters et triesters du type phosphate et les diesters du type phosphite, possédant un chlore la bile, sur une amine, caractérisé en ce qu'il consiste à faire réagir une amine ayant au moins 6 atomes de carbone, pendant au moins 3 heures, avec un réactif du type ester phosphorique de formule

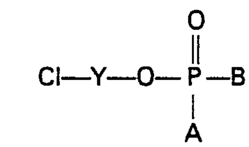

dans laquelle
A est choisi parmi OM et OYCl,
B est choisi parmi H, OM′ et OYCl, à la condition que M′ soit un radical organique,
Y est le radical 2-hydroxypropyle,
M et M′ sont, séparément, un radical organique choisi parmi les radicaux alkyle ou hydroxyalkyle ayant jusqu'à 6 atomes de carbone, dihydroxyalkyle ayant jusqu'à 10 atomes de carbone, glycéryle, cycloalkyle ayant jusqu'à 6 atomes de carbone, M étant en outre l'hydrogène ou le radical salin choisi parmi les métaux alcalins, les métaux alcalino-terreux et la mono-, di- ou triéthanolamine
le réactif du type ester phosphorique ayant un pH compris entre environ 5 et 6 avant l'addition de l'amine, le pH étant maintenu entre pH 7 et 9 après cette addition de l'amine à une température d'au moins 80°C, ce réactif du type ester phosphorique étant préparé à un pH entre 4 et 5 à une température d'au moins 80°C.

2. Procédé selon la revendication 1, caractérisé en ce que l'ester phosphorique a pour formule

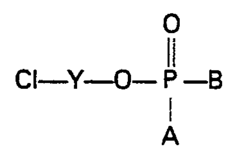

où A est OM, B est OM′, M est Na et M′ est le radical glycéryle.

3. Procédé selon la revendication 1, caractérisé en ce que le réactif du type ester phosphorique a pour formule

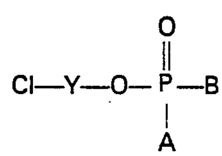

où A est OM, B est OM′, M est le radical glycéryle et M′ est

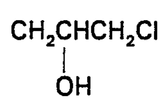

4. Procédé selon la revendication 1, caractérisé en ce que le réactif du type ester phosphorique a pour formule

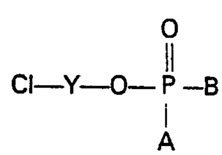

où A est OM, B est H, $M^{\oplus}$ est le radical glycéryle.

5. Procédé selon la revendication 1, caractérisé en ce que le réactif du type ester phosphorique a pour formule

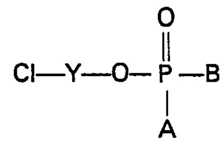

où A est OM, B est H, M est

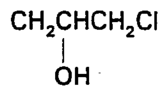

6. Procédé selon la revendication 1, caractérisé en ce que le réactif du type ester phosphorique a pour formule

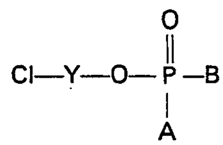

où A et B sont tous deux

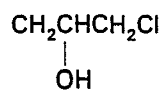

7. Procédé selon la revendication 1, caractérisé en ce que le réactif du type ester phosphorique a pour formule

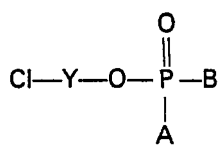

où A est OM, B est OYCl, M est Na.

8. Agent tensioactif contenant du phosphore répondant à la formule:

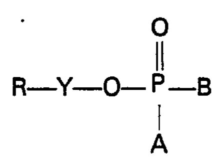

où A est choisi parmi OM et OYCl, B est choisi parmi H, OM', et OYCl à la condition que M' soit un radical organique, Y est le radical 2-hydroxypropyle, R est une amine primaire, secondaire ou tertiaire ayant plus de 6 atomes de carbone, M et M' sont, séparément, un radical organique choisi parmi les radicaux alkyle ou hydroxyalkyle ayant jusqu'à 6 atomes de carbone, dihydroxyalkyle ayant jusqu'à 10 atomes de carbone, glycéryle, cycloalkyle ayant jusqu'à 6 atomes de carbone, M étant en outre l'hydrogène ou un radical salin choisi parmi les métaux alcalins, les métaux alcalino-terreux et la mono-, la di- ou la triéthanolamine, obtenu en faisant réagir un réactif du type ester phosphorique, de formule

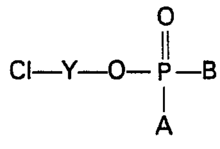

pendant au moins 3 heures, sur une amine R—H, le réactif du type ester phosphorique ayant un pH d'environ 5 à 6 avant l'addition de l'amine, le pH étant maintenu entre 7 et 9 après cette addition d'amine à une température d'au moins 80°C, ce réactif du type ester phosphorique étant préparé à un pH compris entre 4 et 5 à une température d'au moins 80°C.

9. Agent tensioactif contenant du phosphore selon la revendication 8, répondant à la formule

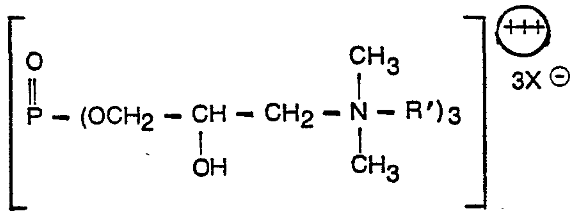

où R′ est un radical amine tertiaire de 6 à 40 atomes de carbone; et X est un anion.

10. Agent tensioactif contenant du phosphore selon la revendication 9, répondant à la formule:

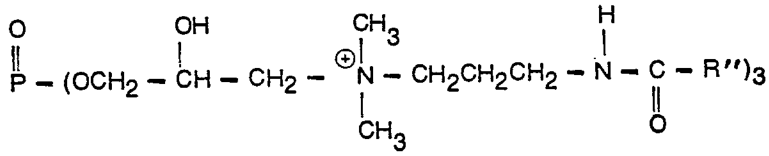

où R″ est un radical alkyle de 7 à 17 atomes de carbone.

11. Agent tensioactif contenant du phosphore selon la revendication 9, répondant à la formule

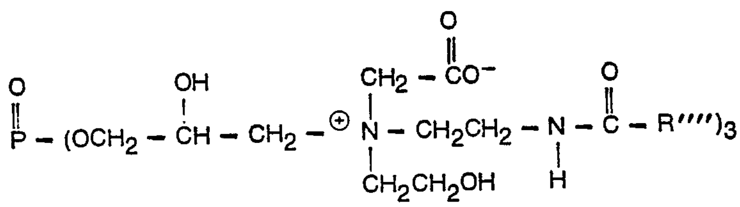

où 70% de R″″ sont un radical alkyle ayant 11 atomes de carbone.

12. Agent tensioactif contenant du phosphore de formule

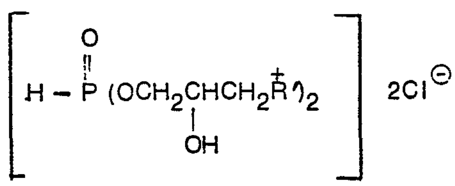

où R′ est un radical ammonium quaternaire.

13. Agent tensioactif contenant du phosphore selon la revendication 12, caractérisé en ce que R′ a pour formule

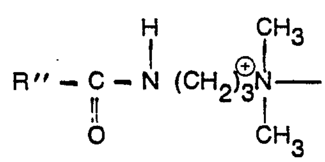

où R″ est un radical alkyle en $C_7$ à $C_{17}$.

14. Agent tensioactif contenant du phosphore selon la revendication 12, dans lequel R′ a pour formule

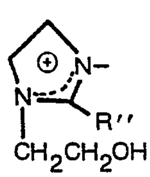

où R″ est un radical alkyle en $C_7$ à $C_{17}$.

15. Agent tensioactif contenant du phosphore de formule

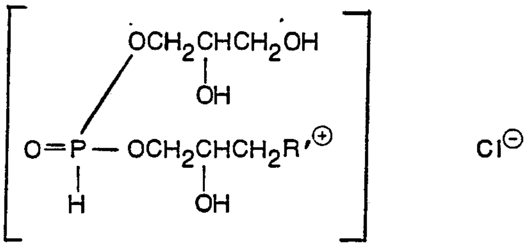

où R est un radical ammonium quaternaire.

16. Agent tensioactif contenant du phosphore selon la revendication 15, caractérisé en ce que R′ a pour formule

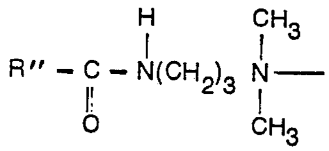

où R″ est un radical alkyle en $C_7$ à $C_{17}$.

17. Agent tensioactif contenant du phosphore selon la revendication 15, caractérisé en ce que R′ a pour formule:

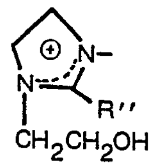

ou R″ est un radical alkyle en $C_7$ à $C_{17}$.

**Patentansprüche**

1. Verfahren zur Herstellung von Phosphor enthaltenden oberflächenaktiven Mitteln durch Reaktion eines Phosphor-Esters ausgewählt aus Phosphat-di- und -triestern und Phosphit-diestern mit einem labilen Chlor als Ausgangsstoff mit einem Amin, gekennzeichnet durch die Umsetzung eines Amins mit wenigstens 6 KohlenstoffAtomen während eines Zeitraums von wenigstens 3 Stunden mit einem Phosphor-Ester-Ausgangsstoff der Formel

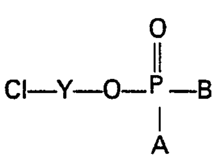

in der

A ausgewählt ist aus OM und OYCl,

B ausgewählt ist aus H, OM′ und OYCl, mit der Maßgabe, daß M′ ein organischer Rest ist,

Y 2-Hydroxypropyl ist,

M und M′ jeweils für sich ein organischer Rest ausgewählt aus Alkyl oder Hydroxyalkyl mit bis zu 6 Kohlenstoff-Atomen, Dihydroxyalkyl mit bis zu 10 Kohlenstoff-Atomen, Glyceryl und Cycloalkyl mit bis zu 6 Kohlenstoff-Atomen sind, wobei M zusätzlich Wasserstoff oder ein Salzrest ausgewählt aus Alkalimetallen, Erdalkalimetallen sowie Mono-, Di- oder Triethanolamin ist,

wobei der Phosphor-Ester-Ausgangsstoff vor dem Amin-Zusatz einen pH von etwa 5 bis 6 besitzt und der pH nach der Amin-Zugabe bei einer Temperatur von wenigstens 80°C auf pH 7 bis 9 gehalten wird, und wobei der Phosphor-Ester-Ausgangsstoff bei einem pH von 4 bis 5 bei einer Temperatur von wenigstens 80°C hergestellt wurde.

2. Verfahren nach Anspruch 1, dadurch gekennzeichnet, daß der Phosphor-Ester die Formel

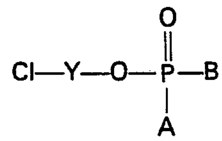

besitzt, in der
A OM ist,
B OM′ ist,
M Na ist und
M′ Glyceryl ist.

3. Verfahren nach Anspruch 1, dadurch gekennzeichnet, daß der Phosphor-Ester die Formel

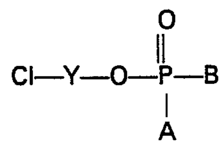

besitzt, in der
A OM ist,
B OM′ ist,
M Glyceryl ist und
M′ 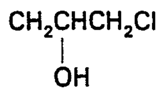 ist.

4. Verfahren nach Anspruch 1, dadurch gekennzeichnet, daß der Phosphor-Ester die Formel

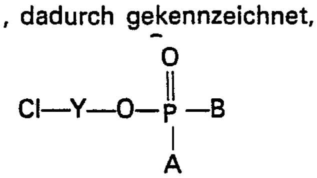

besitzt, in der
A OM ist,
B H ist und
M⊕ Glyceryl ist.

5. Verfahren nach Anspruch 1, dadurch gekennzeichnet, daß der Phosphor-Ester die Formel

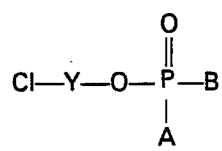

besitzt, in der
A OM ist,
B H ist und
M 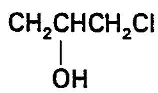 ist

6. Verfahren nach Anspruch 1, dadurch gekennzeichnet, daß der Phosphor-Ester die Formel

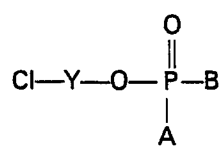

besitzt, in der sowohl A als auch B

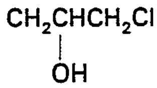 sind

7. Verfahren nach Anspruch 1, dadurch gekennzeichnet, daß der Phosphor-Ester die Formel

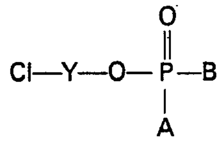

besitzt, in der
A OM ist,
B OYCl ist und
M Na ist.

8. Phosphor enthaltendes oberflächenaktives Mittel mit der Struktur

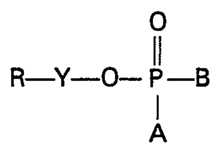

in der
A ausgewählt ist aus OM und OYCl,
B ausgewählt ist aus H, OM′ und OYCl, mit der Maßgabe, daß M′ ein organischer Rest ist,
Y 2-Hydroxypropyl ist,
R ein primäres, sekundäres oder tertiäres Amin mit mehr als sechs Kohlenstoff-Atomen ist,
M und M′ jeweils für sich ein organischer Rest ausgewählt aus Alkyl oder Hydroxyalkyl mit bis zu 6 Kohlenstoff-Atomen, Dihydroxyalkyl mit bis zu 10 Kohlenstoff-Atomen, Glyceryl und Cycloalkyl mit bis zu 6 Kohlenstoff-Atomen sind, wobei M zusätzlich Wasserstoff oder ein Salzrest ausgewählt aus Alkalimetallen, Erdalkalimetallen sowie Mono-, Di- oder Triethanolamin ist, hergestellt durch Umsetzung eines Phosphor-Ester-Ausgangsstoffs der Formel

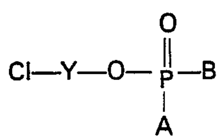

mit einem Amin R—H während eines Zeitraums von wenigstens 3 Stunden, wobei der Phosphor-Ester-Ausgangsstoff vor dem Amin-Zusatz einen pH von etwa 5 bis 6 besitzt und der pH nach der Amin-Zugabe bei einer Temperatur von wenigstens 80°C auf pH 7 bis 9 gehalten wird, und wobei der Phosphor-Ester-Ausgangsstoff bei einem pH von 4 bis 5 bei einer Temperatur von wenigstens 80°C hergestellt wurde.

9. Phosphor enthaltendes oberflächenaktives Mittel nach Anspruch 8 mit der Struktur

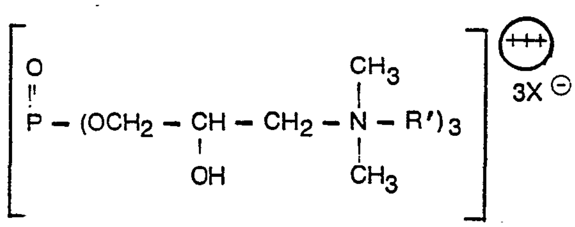

in der
R′ der Rest eines tertiären Amins mit 6 bis 40 Kohlenstoff-Atomen ist und
X ein Anion ist.

10. Phosphor enthaltendes oberflächenaktives Mittel nach Anspruch 9 mit der Struktur

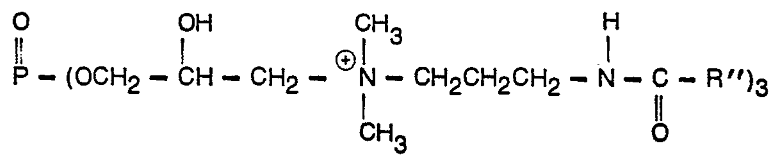

in der

R'' Alkyl mit 7 bis 17 Kohlenstoff-Atomen ist.

11. Phosphor enthaltendes oberflächenaktives Mittel nach Anspruch 9 mit der Struktur

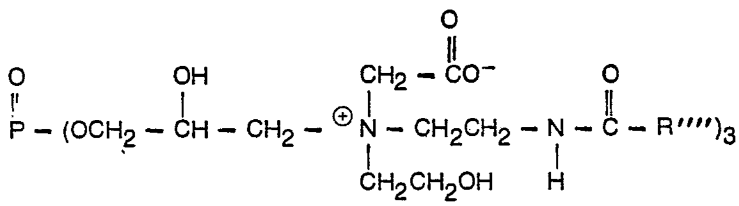

in der 70% von

R'''' Alkyl mit 11 Kohlenstoff-Atomen ist.

12. Phosphor enthaltendes oberflächenaktives Mittel der Formel

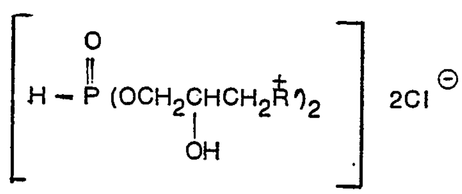

in der

R' ein quaternärer Ammonium-Rest ist.

13. Phosphor enthaltendes oberflächenaktives Mittel nach Anspruch 12, worin

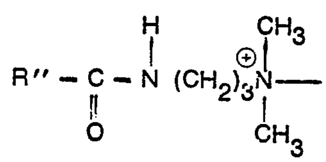

ist und

R'' $C_7$ bis $C_{17}$-Alkyl ist.

14. Phosphor enthaltendes oberflächenaktives Mittel nach Anspruch 12, worin

R'

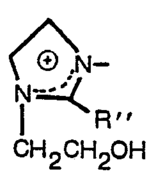

is und

R'' $C_7$- bis $C_{17}$-Alkyl ist.

15. Phosphor enthaltendes oberflächenaktives Mittel der Formel

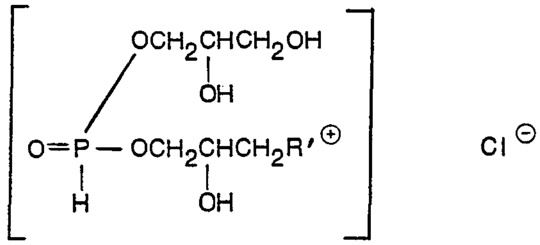

in der
R' ein quaternärer Ammonium-Rest ist.

16. Phosphor enthaltendes oberflächenaktives Mittel nach Anspruch 15, worin R'

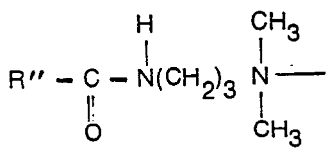

ist und
R'' $C_7$- bis $C_{17}$-Alkyl ist.

17. Phosphor enthaltendes oberflächenaktives Mittel nach Anspruch 15, worin R'

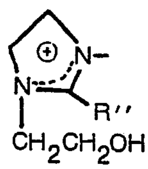

ist und
R'' $C_7$- bis $C_{17}$-Alkyl ist.

FIG. I.

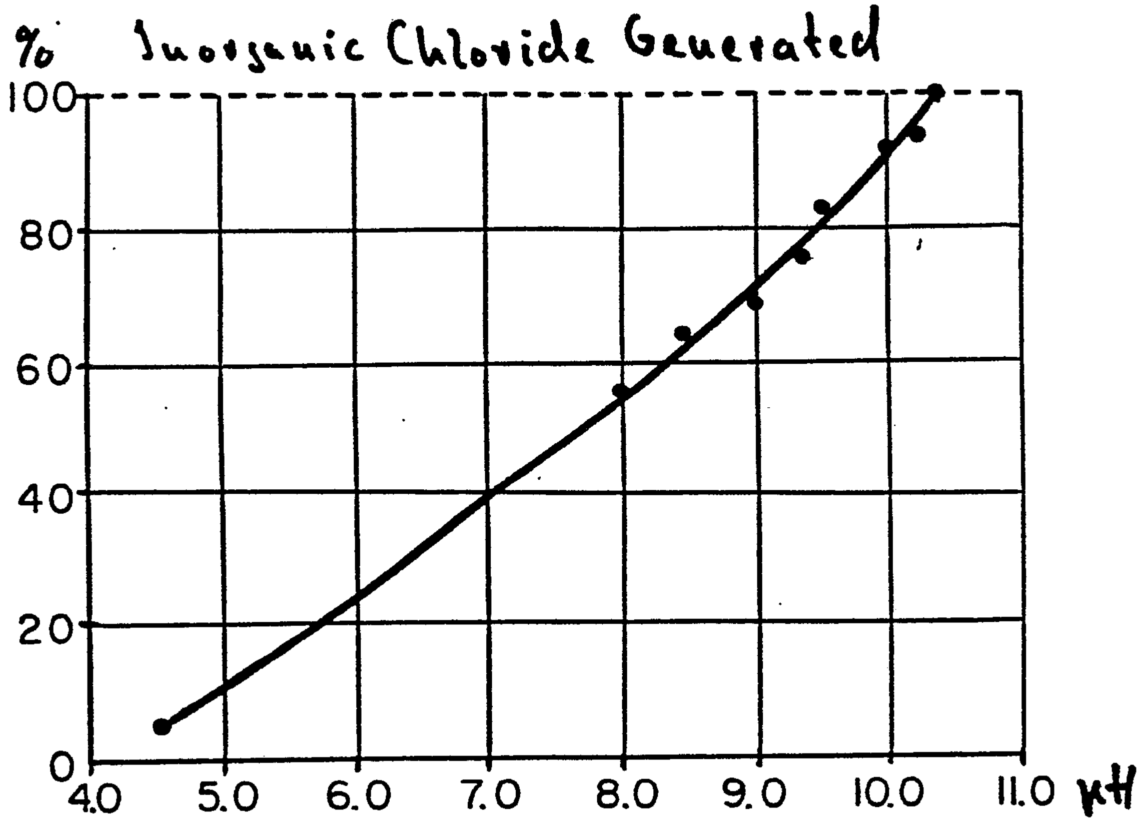


| pH | % Inorganic Chloride |
|---|---|
| 4.6 | 5.5 % |
| 8.0 | 54.0 % |
| 8.5 | 63.0 % |
| 9.0 | 66.2 % |
| 9.4 | 75.0 % |
| 9.5 | 83.6 % |
| 10.0 | 91.0 % |
| 10.2 | 95.0 % |
| 10.3 | 100.0 % |

FIG. 2.

Foam Profile

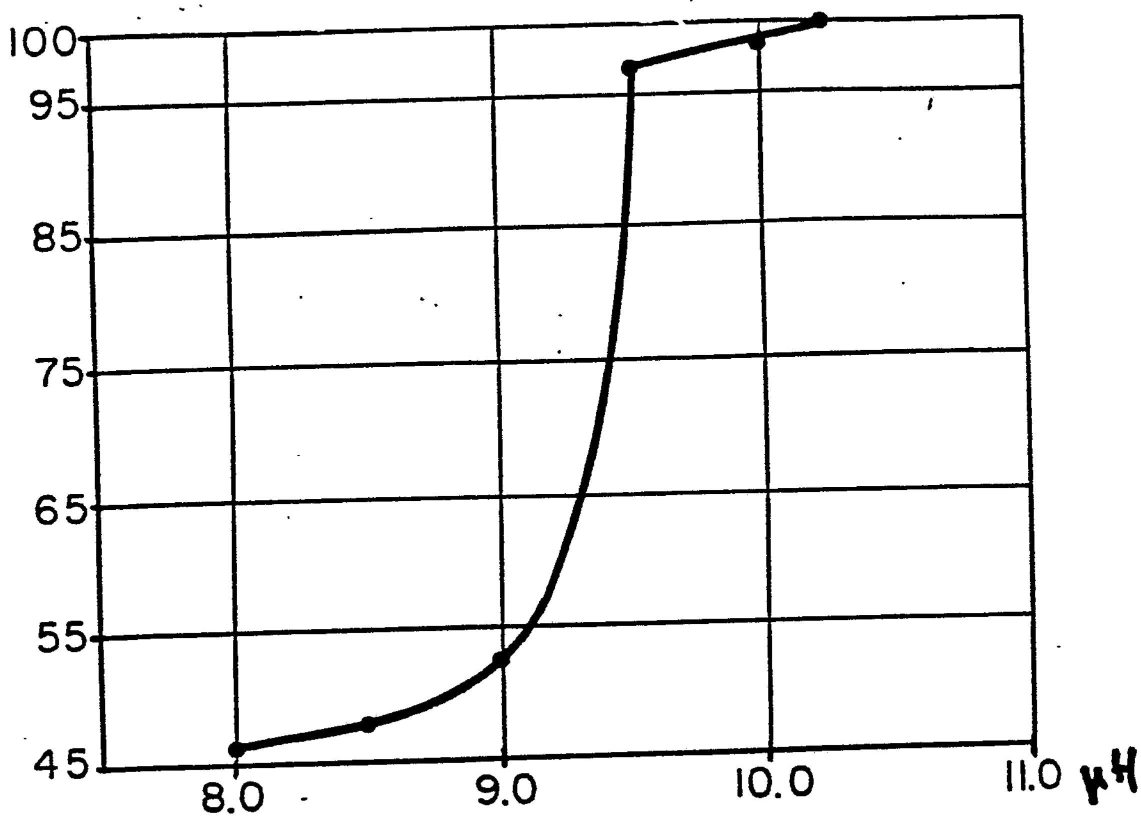


| pH | Foam height |
|---|---|
| 8.0 | 46 |
| 8.5 | 48 |
| 9.0 | 53 |
| 9.5 | 97 |
| 10.0 | 99 |
| 10.2 | 100 |

FIG. 3.

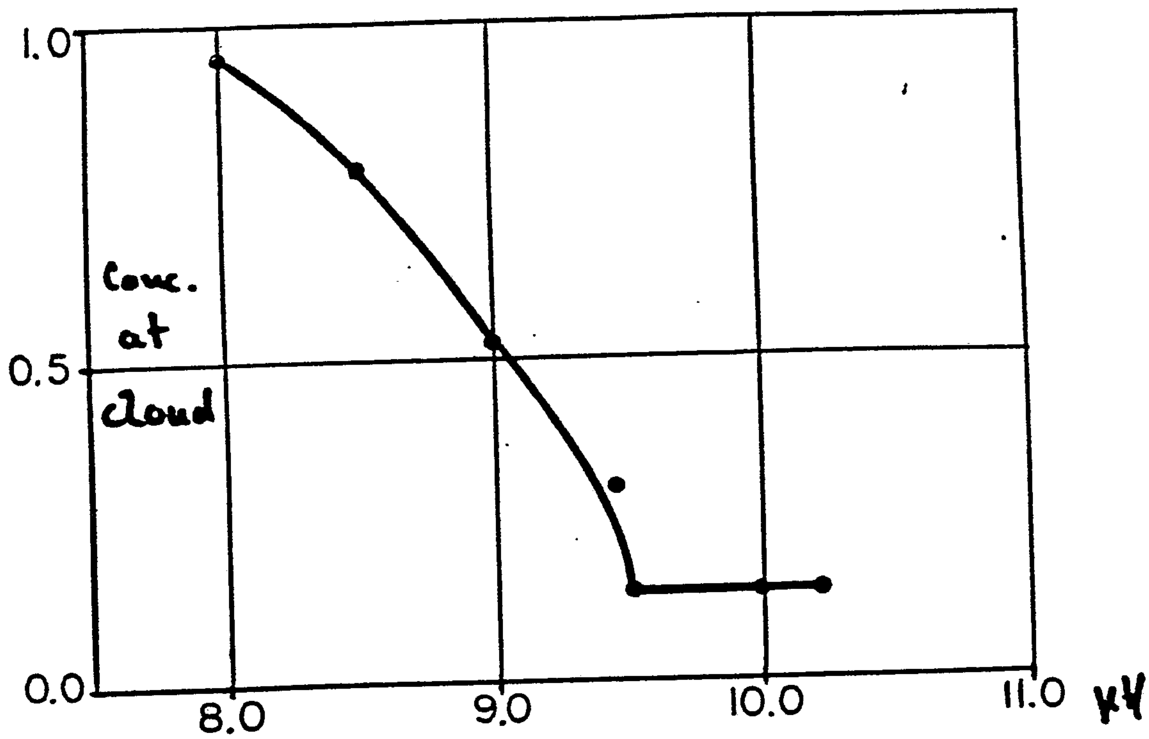


| pH | Concentration at cloud |
|---|---|
| 8.0 | 0.95 % |
| 8.5 | 0.78 % |
| 9.0 | 0.52 % |
| 9.4 | 0.29 % |
| 9.5 | 0.12 % |
| 10.0 | 0.12 % |
| 10.2 | 0.12 % |